**Europäisches Patentamt**

**European Patent Office**

**Office européen des brevets**

(11) Veröffentlichungsnummer : **0 536 199 B1**

(12) **EUROPÄISCHE PATENTSCHRIFT**

(45) Veröffentlichungstag der Patentschrift :
09.02.94 Patentblatt 94/06

(21) Anmeldenummer : 91911471.0

(22) Anmeldetag : 08.06.91

(86) Internationale Anmeldenummer :
PCT/EP91/01077

(87) Internationale Veröffentlichungsnummer :
WO 92/00058 09.01.92 Gazette 92/02

(51) Int. Cl.⁵ : **A61K 7/08,** A61K 7/50,
C11D 1/94

(54) **EXTRA MILDE DUSCHGEL- UND HAARSHAMPOO-FORMULIERUNG MIT NIEDRIGER TENSIDKONZENTRATION.**

(30) Priorität : 27.06.90 DE 4020500

(43) Veröffentlichungstag der Anmeldung :
14.04.93 Patentblatt 93/15

(45) Bekanntmachung des Hinweises auf die
Patenterteilung :
09.02.94 Patentblatt 94/06

(84) Benannte Vertragsstaaten :
AT BE CH DE DK ES FR GB GR IT LI LU NL SE

(56) Entgegenhaltungen :
EP-A- 0 099 987
EP-A- 0 236 677
EP-A- 0 417 501
DE-A- 3 910 652
GB-A- 2 219 594
US-A- 3 990 991

(73) Patentinhaber : **Joh. A. Benckiser GmbH**
**Postfach 21 10 67, Ludwig-Bertram-Strasse 8**
**+ 10**
**D-67059 Ludwigshafen (DE)**

(72) Erfinder : **BAUST, Heinrich**
**Westende 29**
**D-6831 Plankstadt (DE)**
Erfinder : **WÄSCHENBACH, Guido**
**Lüderitzstr. 35**
**D-6800 Mannheim 81 (DE)**

(74) Vertreter : **Grussdorf, Jürgen, Dr.**
**Patentanwälte Zellentin & Partner**
**Rubensstrasse 30**
**D-67061 Ludwigshafen (DE)**

**Beschreibung**

Gegenstand der vorliegenden Erfindung ist eine neue extramilde Duschgel- und Haarshampoo-Formulierung mit niedriger Tensidkonzentration.

Die Produktionsmenge für Duschbäder und Haarshampoos der Kosmetikindustrie in Deutschland liegt derzeit bei ca. 110.000 jato. Der Tensidanteil in den handelsüblichen Produkten bewegt sich dabei zwischen 15 und 25 %. So gelangen ca. 20.000 jato Tenside in die Abwässer, wo sie mehr oder weniger schnell und vollständig abgebaut werden. Eine Verringerung der waschaktiven Substanzen ist sowohl aus ökonomischer als auch ökologischer Sicht ein erstrebenswertes Ziel.

Es ist bekannt, daß bestimmte, so auch ethersulfathaltige Tenside mit stark ausgeprägter Schaumbildung eine drastische Senkung der Waschaktiven-Substanzen von z. B. 20 % auf 10 % zulassen, daher im Gegenzug gegenüber anderen Tensiden jedoch eine geringere Viskosität der Mischung bewirken. Der eintretende Viskositätsabfall muß durch Abwässer belastende-Elektrolytzugaben(NaCl, $Na_2SO_4$) oder zusätzlich das Abwasser belastende organische Verdicker kompensiert werden. Die Salzbelastung übersteigt dabei leicht die Tensid-Konzentration, was einerseits das Risiko des Augenbrennens, andererseits das der Instabilität (Aussalzungseffekt) deutlich erhöht.

Es stellte sich daher die Aufgabe, eine ethersulfathaltige milde und gut schäumende Tensidkombination für die Haut- und Haarpflege zu finden, mit der bei Tensidkonzentrationen von unter 10 % hochviskose Zubereitungen mit einem Minimum an Elektrolytsalzen und ohne zusätzliche Verdicker hergestellt werden können. Die Produkte sollen weiterhin gut biologisch abbaubar sein, um die Umwelt nicht zu belasten.

Überraschenderweise wird diese Aufgabe durch die in den Ansprüchen näher gekennzeichneten Merkmale gelöst, beziehungsweise gefördert.

Aus der älteren nicht vorveröffentlichten Anmeldung P 39 10 652.7 ist bekannt, daß eine Tensidkombination aus Laurylpolyglykolethercarboxylat, Laurylsulfat und Laurinsäureamidopropylbetain mit 2 - 10 % Natriumchlorid zu einem gut verträglichen Duschgel verarbeitet werden kann. Das dabei verwendete Laurylglykolethercarboxylat hatte einen Ethoxylierungsgrad von 8 - 10 Ethoxygruppen. Um ein ausreichendes Schaumverhalten zu erzeugen, war eine Gesamttensid-Konzentration von 15 - 20 % erforderlich. Zur Viskositätseinstellung wurden 4,5 % NaCl zugesetzt.

Aus der EPA 00 99 987 sind Haarbehandlungsmittel bekannt, welche an Schichtsilikate absorbierte Tenside enthalten, und neben einer schonenden Reinigungswirkung die Splißrate und elektrostatische Aufladung der Haare vermindern sollen. Neben den Schichtsilikaten werden noch organische Verdickungsmittel benötigt. Die verdickende Wirkung von speziellen Tensidmischungen ist nicht festgestellt worden.

Aus der US-P 39 90 991 sind ferner Shampoos bekannt, welche eine Mischung aus amphoteren, cryptoanionischen und kationischen Tensiden enthalten. Als anionisches Tensid kann dabei eine Alkylpolyglykolethercarbonsäure mit einem EO-Grad von 6,5 verwendet werden. Als amphoteres Tensid sind neben den bevorzugten Imidazolderivaten auch Fettsäurealkylbetaine einsetzbar. Auch bei diesen Kombinationen muß zur Verdickung ein organischer Verdicker zugesetzt werden. Darüber hinaus ist die Schaumbildung nur mittelmäßig.

Alkylethercarbonsauren mit geringerem Ethoxylierungsgrad (2-5) werden trotz ihrer guten Tensidwirkung in Körperreinigungsmitteln wegen der dabei auftretenden Augen- und Hautirritationen nicht eingesetzt (Seifen, Öle, Wachse (1983) S. 353-355).

Überraschenderweise zeigen die bei alleiniger Verwendung agressiveren Alkyl-ethercarbonsäuren mit niederem Ethoxylierungsgrad (2 - 5) in der erfindungsgemäßen Kombination keine Hautirritationen mehr und weisen ein so starkes Schaumbildungsvermögen auf, daß Gesamttensid-Konzentrationen von unter 10 % möglich sind.

Alkylpolyglykolethercarbonsäuren im Sinne der Erfindung sind Verbindungen der Formel I

$$R\text{-}O\text{-}(CH_2 \text{ - } CH_2 \text{ - } O)_n \text{ - } CH_2 CO_2 H \qquad (I)$$

in der R eine Alkylgruppe mit 8 bis 20 C-Atomen, vorzugsweise 10 - 16, insbesondere 12 - 14 C-Atomen, und n eine natürliche Zahl von 2 - 5, vorzugsweise 2,5 bedeutet. Die Carbonsäuren sind vorzugsweise mit Natrium, Kalium oder Ammoniumionen, insbesondere Tris(hydroxymethyl)aminomethan neutralisiert.

Bei der üblichen Herstellung der Verbindungen I durch Poly-Kondensation von Fettalkoholen mit Ethylenoxid (Ethoxylierung) und folgende Umsetzung mit Monochloressigsäure entstehen Gemische, die für den Einsatz als Tensid nicht oder nur grob getrennt werden. Die Zahl n ist daher als Mittelwert der enthaltenen Verbindungen zu verstehen. Auch gewisse Mengen an nicht umgesetzten Fettalkoholethoxylaten können noch enthalten sein.

Alkylethersulfate im Sinne der Erfindung sind als Tenside gut bekannte von Fettalkoholen abgeleitete, ethoxylierte Monoalkylschwefelsäurester, mit besonders gutem Schaumverhalten unter Hartwasserbedingungen.

Der Ethoxylierungsgrad beträgt im Mittel 2-5. Die Endgruppe ist eine lineare Alkylgruppe mit 8-20, vorzugsweise 10-18 C-Atomen. Lauryl- und CocosfettalkoholGruppen, werden aus wirtschaftlichen Gründen bevorzugt.

Fettsäureamidopropylbetaine sind ebenfalls als Tenside gut bekannt. Als Fettsäuren sind solche mit 8 - 20, insbesondere 12 - 14 C-Atomen üblich. Wegen des optimalen Schaumverhaltens und der Zusammenwirkung mit den beiden anderen Komponenten im Sinne einer guten Verdickung, ohne daß bei tieferen Temperaturen ein schwerlöslicher Niederschlag entsteht, wird Laurylamidopropylbetain bevorzugt.

Ein weiterer Vorteil der erfindungsgemäßen Mischungen liegt darin, daß sie eiweißfrei formuliert werden können, da auch die hierbei bekannten Aminosäurengemische allergen sein können. Als Rückfettungskomponente wird die Verwendung von Laurinsäuremonoglycerid zusammen mit der erfindungsgemäßen Tensidmischung vorgeschlagen.

Zur Solubilisierung von üblicherweise in derartigen Hautreinigungsmitteln eingesetzten Parfümölen hat sich der Einsatz von Laurylalkoholethoxylat besonders bewährt, da dieses Mittel Öl besonders gut löslich macht.

Ein Duschgel, wie es bevorzugt mit Hilfe der vorgenannten Substanzen herstellbar ist, enthält neben dem schon genannten Parfümöl Kochsalz als Verdickungsmittel und/oder den polyfunktionalen Wirkstoff Na-Hexametaphosphat. Des weiteren Substanzen zur Konservierung, z.B. Dibromcyanobutan/Phenoxyethanol (Euxyl K 400, Wz der Fa. Schuhlke u. Mayr) und Wasser.

Insbesondere in Haarshampoos werden zur Verbesserung der Eigenschaften noch Conditionierer, insbesondere quarternierte Copolymere wie Dimethylalkylammoniumchloridpolymer (Merquat 550, Wz der Fa. Merck &Co. Inc.), Vinylpyrrolidon/ Dimethylaminoethylmethacrylat (Gafquad 734 - Wz der General Aniline Film Co.) oder Miropol A 15 (Wz der Miranal Chem. Co. Inc.) in Mengen bis zu 2 % insbesondere 0,2 - 1 % eingesetzt.

Erfindungsgemäß zusammengesetzte Duschgele weisen vorzugsweise die nachstehenden Mengenverhältnisse (Gew. %) auf:

| Alkylpolyglycolether-carboxylat | 2,0 | - | 5 |
| Fettalkoholethersulfat | 2,0 | - | 7 |
| Fettsäure-amidopropyl-betain | 1,0 | - | 3 |
| Eiweißhydrolysat | 0,3 | - | 1,5 |
| Laurylalkohol-ethoxylat | 0,1 | - | 0,5 |
| Conditionierer | 0 | - | 2 |
| Parfüm | 0,5 | - | 2 |
| Na-Hexametaphosphat | 0 | - | 5 |
| NaCl | 2 | - | 10 |
| Konservierungsmittel | 0,05 | - | 0,5 |
| Wasser | | ad 100 | |

Ausführungsbeispiele

Beispiel 1        10 % WAS-Duschgel

                                                                                    E %


```
Cl2/14 Ethercarbonsäure, 2,5 Mol EO, Na-Salz,   4,27
Natriumlauryldiglykolethersulfat                3,25
Laurylsäure-amidopropylbetain,                   2,53
Parfümöl                                         0,72
Konservierungsmittel (Euxyl K 400 *)             0,07
NaCl                                      ca.    2,00
Wasser, entkeimt                          ad     100
```

```
* Wz der Fa. Schühlke u. Mayr
pH-Wert mit NaOH bzw. TRIS auf pH 6,5 - 6,8 eingestellt
```

Beispiel 2        8 % WAS-Duschgel

                                                                                    E %

```
C 12/14 Ethercarbonsäure, 2,5 Mol EO  Na-Salz,   3,39
C 12/14 Alkylethersulfat, 3,5 Mol EO  Na-Salz,   2,58
C 12/14 Fettsäure-amidopropylbetain              2,01
Eiweißhydrolysat                                 0,72
Glycerinpartialester, ethoxyliert               0,26
Parfümöl                                         0,57
Farbstoffe                                       q.s.
Konservierungsmittel                             0,07
NaCl                                      ca.    2,00
Wasser, entkeimt                          ad     100
```

```
pH-Wert mit NaOH bzw. TRIS auf pH 6,5 - 6,8 eingestellt
```

4

Beispiel 3      10 % WAS-Haarshampoo

|  | E % |
|---|---|
| Laurylethercarbonsäure, 2,5 Mol EO (Na-Salz) | 4,27 |
| Fettalkoholethersulfat * | 3,25 |
| Fettsäure-amidopropylbetain * | 2,53 |
| Eiweißhydrolysat | 0,91 |
| Glycerinpartialester, ethoxyliert | 0,32 |
| Parfümöl | 0,50 |
| Farbstoffe | q.s. |
| Konservierungsmittel | 0,07 |
| NaCl | ca.  2,00 |
| Wasser, entkeimt | ad  100 |

(*R = Cocos-' bzw. Lauryl-)


pH-Wert mit NaOH bzw. TRIS auf pH 6,5 - 6,8 eingestellt


Beispiel 4      8% WAS-Haarshampoo


|  | E % |
|---|---|
| — | |
| C 12/14 Ethercarbonsäure, 2,5 Mol EO  Na-Salz | 3,32 |
| Fettalkoholethersulfat * | 2,58 |
| Fettsäure-amidopropylbetain * | 2,01 |
| Eiweißhydrolysat | 0,72 |
| Konservierungsmittel | 0,07 |
| Glycerinpartialester, ethoxyliert | 0,26 |
| Parfümöl | 0,57 |
| Farbstoffe | q.s. |
| Euxyl K 400 | 0,07 |
| NaCl | ca.  2,00 |
| Wasser, entkeimt | ad  100 |


(* R = Cocos-bzw. Lauryl-)


pH-Wert mit NaOH bzw. TRIS auf pH 6,5 - 6,8 eingestellt

EP 0 536 199 B1

Beispiel 5        10 % WAS-Duschgel

E %

C 12/14 Fettalkoholethersulfat, 2 Mol EO, Na-Salz   6,25

C12/14 Ethercarbonsäure, 2,5 Mol EO, Na-Salz   2,41

Fettsäure-amidopropylbetain*   1,19

Eiweißhydrolysat   0,44

Glycerinpartialester, ethoxyliert   0,28

Parfümöl   0,75

Diglykolstearat**   ca. 0,50

Farbstoffe   q.s.

Konservierungsmittel   0,07

Na Cl   ca. 2,65

Wasser, entkeimt   ad 100

*   ( R = Cocos- bzw. Lauryl-)

pH-Wert mit NaOH bzw. TRIS auf pH 6,5-6,8 eingestellt

**für Perlglanz-Produkte

**Patentansprüche**

1.  Duschgel und Haarshampoo welche eine hochviskose, wässrige Lösung einer neutralisierten Tensidkombination aus Alkylpolyglykolethercarboxylat, Fettalkoholethersulfat, und Fettsäure-amidopropyl-betain sowie übliche Hilfs- und Trägerstoffe enthalten dadurch gekennzeichnet, daß die verwendete Alkylpolyglykolethercarbonsäure die allgemeine Formel I aufweist,
    $$R\text{-}O\text{-}(CH_2CH_2O)_n \, CH_2COOH \qquad (I)$$
    in der R eine geradkettige Alkylgruppe mit 8 - 20 C-Atomen und n im Mittel 2 bis 5 bedeutet und in einer Menge von 2-5 Gew.-% enthalten ist, das Fettalkoholethersulfat in einer Menge von 2-7 Gew.-%, das Fettsäureamidopropyl-betain in einer Menge von 1-3 Gew.-%, wobei die Gesamtkonzentration an Tensiden unter 10 Gew.-% beträgt, Elektrolyte in einer Menge von 2-10 Gew.-% enthalten sind und die Mischung frei von sonstigen organischen und anorganischen Verdickern ist.

2.  Duschgel und Haarshampoo nach Anspruch 1, **dadurch gekennzeichnet**, daß der mittlere Ethoxylierungsgrad der Alkylpolyglykolethercarboxylate 2,5 beträgt.

3.  Duschgel und Haarshampoo nach Anspruch 1 und 2, **dadurch gekennzeichnet**, daß das Mittel einen pH-Wert von 5,5 - 7,5 aufweist und als Neutralisierungsmittel KOH, NaOH, vorzugsweise mit Tris(hydroxymethyl)-aminomethan enthalten ist.

4.  Duschgel und Haarshampoo nach einem oder mehreren der Ansprüche 1 - 3, **dadurch gekennzeichnet**, daß das Mengenverhältnis von Fettalkoholethersulfat zu Alkylpolyglykolethercarboxylat 1 : 0,25 bis 1,5,und von Fettalkoholethersulfat zu Fettsäure-amidopropyl-betain wie 1:0,15 bis 1,0 ist.

5.  Duschgel und Haarshampoo nach einem oder mehreren der Ansprüche 1 - 4, **dadurch gekennzeichnet**,

6

EP 0 536 199 B1

daß die Gesamt-Tensidkonzentration bei 10% oder darunter liegt.

6. Duschgel und Haarshampoo nach einem oder mehreren der Ansprüche 1 - 5, **gekennzeichnet** durch einen Gehalt an Laurylalkohol-ethoxylat.

7. Duschgel und Haarshampoo nach einem oder mehreren der Ansprüche 1 - 6, **dadurch gekennzeichnet**, daß Conditionierer, Eiweißhydrolysate und/oder Acrylamid-Polymere enthalten sind.

8. Duschgel und Haarshampoo nach einem oder mehreren der Ansprüchen 1 - 7, **dadurch gekennzeichnet**, daß ein Elektrolyt, insbesondere Natriumchlorid, oder ein Polyphosphat, insbesondere Na-Hexametaphosphat als Verdicker enthalten ist.

9. Duschgel und Haarshampoo nach einem oder mehreren der Ansprüche 1 - 8, **gekennzeichnet** durch folgende Zusammensetzung in Gew. %

| | | | |
|---|---|---|---|
| Alkylpolyglycolether-carboxylat | 2 | – | 5 |
| Fettalkoholethersulfat | 2 | – | 7 |
| Fettsäure-amidopropyl-betain | 1 | – | 3 |
| Eiweißhydrolysat | 0,3 | – | 1,5 |
| Laurylalkohol-ethoxylat | 0,1 | – | 0,5 |
| Conditionierer | 0 | – | 2 |
| Parfüm | 0,5 | – | 2 |
| Na-Hexametaphosphat | 0 | – | 5 |
| NaCl | 2 | – | 10 |
| Konservierungsmittel | 0,05 | – | 0,5 |
| Wasser | ad 100 | | |

## Claims

1. Shower gel and hair shampoo which contains a highly viscous, aqueous solution of a neutralised tenside combination of alkyl polyglycol ether carboxylate, fatty alcohol ether sulphate and fatty acid amidopropylbetaine, as well as usual adjuvant and carrier materials, characterised in that the alkyl polyglycol ether carboxylic acid used has the general formula I,

$$R-O-(CH_2CH_2O)_nCH_2COOH \qquad (I)$$

in which R signifies a straight-chained alkyl group with 8 - 20 C-atoms and n on average 2 to 5, and is contained in an amount of 2 - 5 wt.%, the fatty alcohol ether sulphate in an amount of 2 - 7 Wt.%, the fatty acid amidopropylbetaine in an amount of 1 - 3 wt.%, whereby the total concentration of tensides amounts to below 10 wt.%, electrolytes are contained in an amount of 2 - 10 wt.% and the mixture is free from other organic and inorganic thickeners.

2. Shower gel and hair shampoo according to claim 1, characterised in that the average degree of ethoxylation of the alkyl polyglycol ether carboxylates amounts to 2.5.

3. Shower gel and hair shampoo according to claim 1 and 2, characterised in that the agent has a pH value of 5.5 - 7.5 and, as neutralisation agent, contains KOH, NaOH, preferably tris-(hydroxymethyl)-amino methane.

4. Shower gel and hair shampoo according to one or more of claims 1 - 3, characterised in that the amount ratio of fatty alcohol ether sulphate to alkyl polyglycol ether carboxylate is 1:0.25 to 1.5 and of fatty alcohol ether sulphate to fatty acid amidopropylbetaine is 1:0.15 to 1.0.

7

5. Shower gel and hair shampoo according to one or more of claims 1 - 4, characterised in that the total tenside concentration lies at 10% or below.

6. Shower gel and hair shampoo according to one or more of claims 1 - 5, characterised by a content of lauryl alcohol ethoxylate.

7. Shower gel and hair shampoo according to one or more of claims 1 - 6, characterised in that conditioners, protein hydrolysates and/or acrylamide polymers are presents.

8. Shower gel and hair shampoo according to one or more of claims 1 - 7, characterised in that an electrolyte, especially sodium chloride, or a polyphosphate, especially Na hexametaphosphate, is present as thickener.

9. Shower gel and hair shampoo according to one or more of claims 1 - 8, characterised by the following composition in wt.%

```
alkyl polyglycol ether carboxylate      2 - 5
fatty alcohol ether sulphate            2 - 7
fatty acid amidopropylbetaine           1 - 3
protein hydrolysate                   0.3 - 1.5
lauryl alcohol ethoxylate             0.1 - 0.5
conditioner                             0 - 2
perfume                               0.5 - 2
Na hexametaphosphate                    0 - 5
NaCl                                    2 - 10
preserving agent                     0.05 - 0.5
water                                  ad 100
```

**Revendications**

1. Gel de douche et shampooing, contenant une solution aqueuse, hautement visqueuse, d'une combinaison tensioactive neutralisée à base de carboxylate d'éther d'alkylpolyglycol, d'éthersulfate d'alcool gras et d'amidopropylbétaïne d'acide gras ainsi que des adjuvants et supports courants, caractérisé en ce que l'acide alkylpolyglycoléthercarboxylique utilisé présente la formule générale I

$$R\text{-}O\text{-}(CH_2CH_2O)_nCH_2COOH \qquad (I)$$

dans laquelle R représente un groupe alkyle linéaire ayant 8 à 20 atomes de C et n représente en moyenne 2 à 5, en ce qu'il est contenu en une quantité de 2 à 5 % en poids, l'éthersulfate d'alcool gras étant contenu en une quantité de 2 à 7 % en poids, l'amidopropylbétaïne d'acide gras en une quantité de 1 à 3 % en poids, la concentration globale d'agents tensioactifs étant inférieure à 10 % en poids, des électrolytes étant contenus en une quantité de 2 à 10 % en poids, et en ce que le mélange est exempt d'autres épaississants organiques et inorganiques.

2. Gel de douche et shampooing suivant la revendication 1, caractérisé en ce que le degré d'éthoxylation moyen des carboxylates d'éther d'alkylpolglycol est de 2,5.

3. Gel de douche et shampooing suivant l'une des revendications 1 et 2, caractérisé en ce que le produit présente une valeur de pH de 5,5 à 7,5 et en ce qu'il contient, comme agent de neutralisation, KOH, NaOH, de préférence avec du tris-(hydroxyméthyl)-amino-méthane.

4. Gel de douche et shampooing suivant une ou plusieurs des revendications 1 à 3, caractérisé en ce que le rapport entre l'éthersulfate d'alcool gras et le carboxylate d'éther d'alkylpolyglycol est de 1/0,25 à 1,5 et entre l'éthersulfate d'alcool gras et l'amido-propylbétaïne d'acide gras de 1/0,15 à 1,0.

5. Gel de douche et shampooing suivant une ou plusieurs des revendications 1 à 4, caractérisé en ce que la concentration globale en agents tensioactifs est de 10 % ou moins.

6. Gel de douche et shampooing suivant une ou plusieurs des revendications 1 à 5, caractérisé par une teneur en éthoxylate d'alcool laurylique.

7. Gel de douche et shampooing suivant une ou plusieurs des revendications 1 à 6, caractérisé en ce que des agents de conditionnement, des hydrolysats de protides et/ou des polymères d'acrylamide sont contenus.

8. Gel de douche et shampooing suivant une ou plusieurs des revendications 1 à 7, caractérisé en ce qu'un électrolyte, en particulier du chlorure de sodium, ou un polyphosphate, en particulier de l'hexamétaphosphate de Na, est contenu comme épaississant.

9. Gel de douche et shampooing suivant une ou plusieurs des revendications 1 à 8, caractérisé par la composition suivante, en % en poids :

```
Carboxylate d'éther d'alkylpolyglycol    2    -    5
Ethersulfate d'alcool gras               2    -    7
Amidopropylbétaïne d'acide gras          1    -    3
Hydrolysat de protide                    0,3  -    1,5
Ethoxylate d'alcool laurylique           0,1  -    0,5
Agent de conditionnement                 0    -    2
Parfum                                    0,5  -    2
Hexamétaphosphate de Na                  0    -    5
NaCl                                      2    -   10
Agent de conservation                    0,05 -    0,5
Eau                                          ad 100
```